# EUROPEAN PATENT SPECIFICATION

(11) **EP 0 629 412 B1**
(45) Date of publication and mention of the grant of the patent: **14.01.1998**
(21) Application number: 94105896.8
(22) Date of filing: 15.04.1994
(51) Int. Cl.: A61M 1/10, A61M 1/12, F16C 33/10

(54) **Auxiliary artificial heart embedded in a ventricle of a heart**
Künstliches, in einen Herzventrikel implantiertes Hilfsherz
Coeur artificiel auxiliaire implanté dans un ventricule du coeur

(30) Priority: 03.06.1993 JP 157993/93
(43) Date of publication of application: 21.12.1994
(73) Proprietor: SUN MEDICAL TECHNOLOGY RESEARCH CORPORATION, Suwa-shi, Nagano-ken (JP); SEIKO EPSON CORPORATION, Shinjuku-ku Tokyo 160 (JP)
(72) Inventor: Yamazaki, Kenji, Koganei-shi, Tokyo (JP); Mori, Toshio, Chino-shi, Nagano-ken (JP); Iiyama, Haruo, Chino-shi, Nagano-ken (JP); Yamazaki, Shunichi, Suwa-shi, Nagano-ken (JP); Ito, Nobutaka, Chino-shi, Nagano-ken (JP); Miyao, Osami, Ebina-shi, Kanagawa-ken (JP); Hori, Masanori, Hatano-shi, Kanagawa-ken (JP); Iwasaki, Yukio, Suwa-shi, Nagano-ken (JP); Adachi, Hitoshi, Suwa-gun, Nagano-ken (JP)
(74) Representative: Weber, Joachim, Dr.

(56) References cited:
- EP-A- 0 240 674
- EP-A- 0 410 293
- EP-A- 0 445 782
- US-A- 4 927 407
- US-A- 5 118 264

## Description

This invention relates to an artificial heart, and more particularly an auxiliary artificial heart of an embedded type, embedded in the left or right ventricle of the heart in a human body, and operated at high reliability by preventing body fluids such as blood from adversely entering the artificial heart.

Conventional artificial hearts are of a diaphragm type, of a sack type, of an axially symmetric type, of a centrifugal type, of a pusher plate type or the like. Each of these conventional artificial hearts delivers blood in place of a human heart or by bypassing it.

Recently, there has been developed an auxiliary artificial heart which is embedded in a ventricle of a human heart and has the tip end of a nozzle passing through an aorta valve or the like such that blood is delivered from the ventricle into an aorta through the nozzle. The artificial heart has such a structure that it does not suppress any function of the human heart when it is provided in the human heart and it delivers additional blood into the aorta when blood pumped out from the human heart due to its beats is insufficient. The blood is delivered not only by the artificial heart but also by the pulsing human heart due to its beats. Even if the operation of the artificial heart happens to stop, blood is delivered by the human heart due to its beats.

Naturally, the volume of the part of the artificial heart which is inserted in a ventricle of the human heart must be smaller than the volume of the human heart when it is contracted most. Such an artificial heart has a pump body comprising a cylindrical axial-flow pump section, a nozzle section provided on its distal end and a driving section provided on the proximal end of the axial-flow pump section. The cardiac apex of a ventricle of a human heart is cut and a short cylindrical cardiac apex ring is embedded therein. The pump section and the nozzle section are inserted in the ventricle through the cardiac apex ring, and the distal end of the nozzle section is inserted in an aorta through its aorta valve or the like. The driving section which has a large volume is embedded in a portion of the thorax which is outside of the human heart.

The artificial heart has the following problem in connection with the function of a shaft-sealing mechanism provided between the pump section and the driving section. With the artificial heart, a motor and other elements are housed in the driving section, and the rotor of the pump section is driven via driving shaft extending from the driving section to the pump section. Blood applied with a blood pressure flows through the pump section. In this state, blood is not allowed to enter the space in the driving section. If blood enters the space defined in the driving section, coagulation of blood occurs and the operation of the motor stops.

It is necessary to provide, between the driving section and the pump section, a sealing mechanism for sealing the driving shaft in liquid tight state in order to prevent blood from entering the interior of the driving section. Since, however, the artificial heart is embedded in a human body, the artificial heart must be operated for a long time without maintenance. It is not easy with the present technology to provide a shaft-sealing mechanism with which perfect sealing is maintained for a long time.

EP-A1-445782, which is used as a basis for the preamble of claim 1, refers to an auxiliary artificial heart comprising a pump section and a drive section. The drive section is connected with a driving shaft. In order to prevent blood from entering that drive section, the bearing of the driving shaft is provided with a seal.

US-A-4,927,407 describes a cardiac assist pump. This artificial heart is provided with an infusion pump for supplying a saline solution at a constant fluid flow rate to the rotor chamber of the cardiac assist pump. The solution leaves the rotor chamber by perfusing into the impeller chamber along the interface between the rotor shaft and a lip seal.

The object of this invention is to provide an artificial heart which has a shaft-sealing mechanism for completely preventing blood from entering the interior of a driving section for a long time and which does not need an infusion pump.

According to the invention, the object is solved by the features of the main claim. The sub-claims contain further preferred developments of the invention.

An auxiliary artificial heart according to this invention inserted in a ventricle of a human heart, including a cylindrical cardiac apex ring embedded in the cardiac apex of the human heart by cutting the cardiac apex, and a main body of the artificial heart comprising a cylindrical axial-flow pump section inserted in the ventricle of the human heart through the cardiac apex ring, a nozzle section extending outward from the distal end of the pump section through the aorta valve of the human heart and a driving section provided on the proximal end of the pump section and disposed outside (or externally) of the human heart, for driving the pump section through a driving shaft.

Between the driving section and the pump section is provided a sealing mechanism for maintaining the driving shaft in a liquid tight state so as to prevent blood from entering the interior of the driving section from the pump section. The sealing mechanism defines a sealing liquid chamber surrounding the driving shaft at the driving section and a sealing liquid is filled in the sealing liquid chamber.

According to a preferred embodiment, the sealing liquid includes a physiological sodium chloride solution or an anticoagulant such as heparin. The sealing liquid chamber communicates with a sealing liquid bag made of flexible material, filled with the sealing liquid and embedded in the human body.

The sealing mechanism is provided with an oil seal made of elastic material, closely fitted on the outer peripheral surface of the driving shaft due to its elastic deformation and forming a lubricating film of the sealing liquid between the peripheral surface of the driving shaft and the oil seal.

The pump section is driven by a motor or the like driving unit housed in the driving section. The pump section sucks blood from a ventricle of a human heart and discharges it into an aorta from the nozzle section of the distal end of the pump section by bypassing the aorta valve or the like. Thus, blood is delivered to the aorta not only by the beats of the human heart but also by means of the artificial heart. The artificial heart supplements insufficient amount of blood which is not provided by the human heart, whereby it is ensured that the necessary amount of blood can be delivered. The volume of the pump section is smaller than the volume of the ventricle of the human heart when it contract most so as not to prevent natural beats of the human heart.

The sealing mechanism prevents blood from entering the driving section from the pump section. In this case, the sealing mechanism defines a sealing liquid chamber at the driving section, and a sealing liquid such as a physiological sodium chloride solution is filled in the sealing liquid chamber. Sealing and lubrication of the sealing mechanism are ensured by the sealing liquid, and blood is securely prevented from entering the interior of the driving section from the sealing mechanism.

Even if the sealing mechanism is deteriorated and blood enters the mechanism, the blood which has entered the mechanism is mixed with the sealing liquid. Thus, blood is not coagulated and does not prevent the smooth operation of the artificial heart.

In the embodiment, the sealing mechanism is provided with an oil seal which forms a lubricant film of the sealing liquid between the oil seal and the outer peripheral surface of the driving shaft and is elastically closely fitted on the outer peripheral surface of the driving shaft for securely preventing the entrance of blood such that the oil seal is not worn and has high durability. The oil seal can be designed such that the lubricant film formed between the oil seal and the outer peripheral surface of the driving shaft delivers blood in only one direction toward the pump section. This structure ensures to prevent blood from entering the interior of the driving section.

The sealing liquid chamber communicates with a sealing liquid bag embedded in the human thorax or the like. A sealing liquid is supplemented from the sealing liquid bag and thus can be supplied to the sealing liquid chamber for a long time.

The driving shaft uses a dynamic pressure bearing made of ceramic material operated in the sealing liquid. A coating film is formed between the sliding surfaces due to the dynamic pressure of the sealing liquid, thereby reducing rotational resistance of the bearing and preventing its wear, leading to high reliability.

When the driving shaft is rotated, the dynamic pressure bearing generates dynamic pressure. The dynamic pressure can provide a liquid seal between the sealing liquid chamber and the driving section. The sealing liquid is thereby prevented from flowing from the sealing liquid chamber into the driving section.

The dynamic pressure bearing, which according to the invention is mounted on the distal end portion of the driving shaft, supplies the sealing liquid to the oil seal. Hence, the sealing liquid circulates in the artificial heart, preventing foreign body from depositing.

In a further preferred embodiment, a metal plating is formed on the outer peripheral surface of the driving shaft. Tetrafluoroethylene and its derivatives are made eutectic in the metal plating film, thereby improving not only lubricating properties between the driving shaft and the oil seal but also durability.

The metal plating film of this kind is water-repellent and is well compatible with living body. Any component that contacts blood or the like may be covered entirely with such a metal plating film.

This invention can be more fully understood from the following detailed description when taken in conjunction with the accompanying drawings, in which:
FIG. 1 is a cross sectional view of an artificial heart of a first embodiment of this invention, provided in the left ventricle of a human heart;
FIG. 2 is a longitudinal cross-sectional view of the main body of the artificial heart of the first embodiment;
FIG. 3 is a cross sectional view of a sealing section;
FIG. 4 is an enlarged cross-sectional view showing the structure of a film formed on the outer peripheral surface of a driving shaft;
FIG. 5 is an exploded view of a dynamic pressure bearing at the proximal end portion of a driving shaft;
FIG. 6 is an exploded perspective view of a dynamic pressure bearing;
FIG. 7 is a diagram showing the dynamic pressure bearing, as viewed in the direction of arrows 7 in FIG. 5;
FIG. 8 is a diagram showing the dynamic pressure bearing, as viewed in the direction of arrows 8 in FIG. 5;
FIG. 9 is a sectional view taken along line 9 in FIG. 8;
FIG. 10 is a longitudinal cross-sectional view of an embodiment of the dynamic pressure bearing at the distal end portion of a driving shaft;
FIG. 11 is a longitudinal cross-sectional view of a further dynamic pressure bearing;
FIG. 12 is a longitudinal cross-sectional view of the main body of an artificial heart of a second embodiment according to this invention;
FIG. 13 is a longitudinal cross-sectional view of the main body of the artificial heart of a third embodiment according to this invention; and
FIG. 14 is an enlarged longitudinal cross-sectional view of part of FIG. 13.

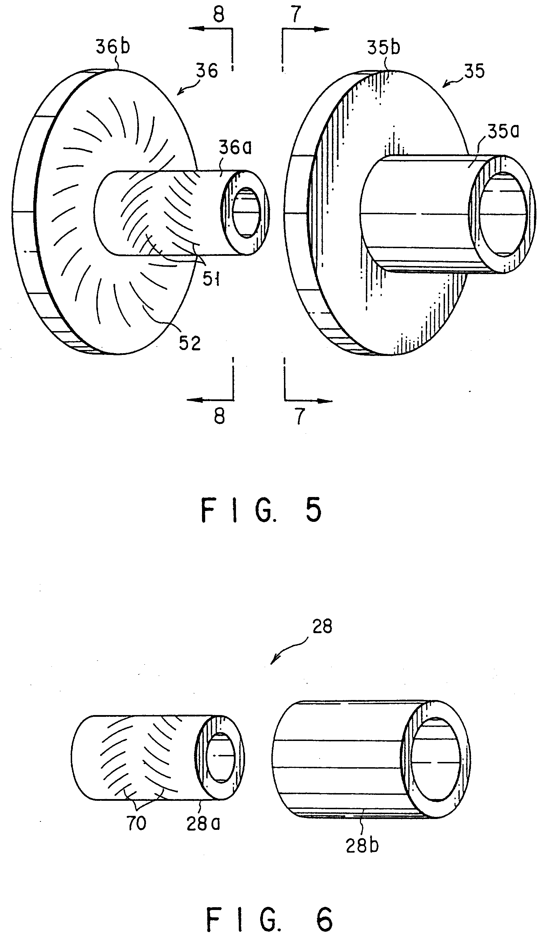

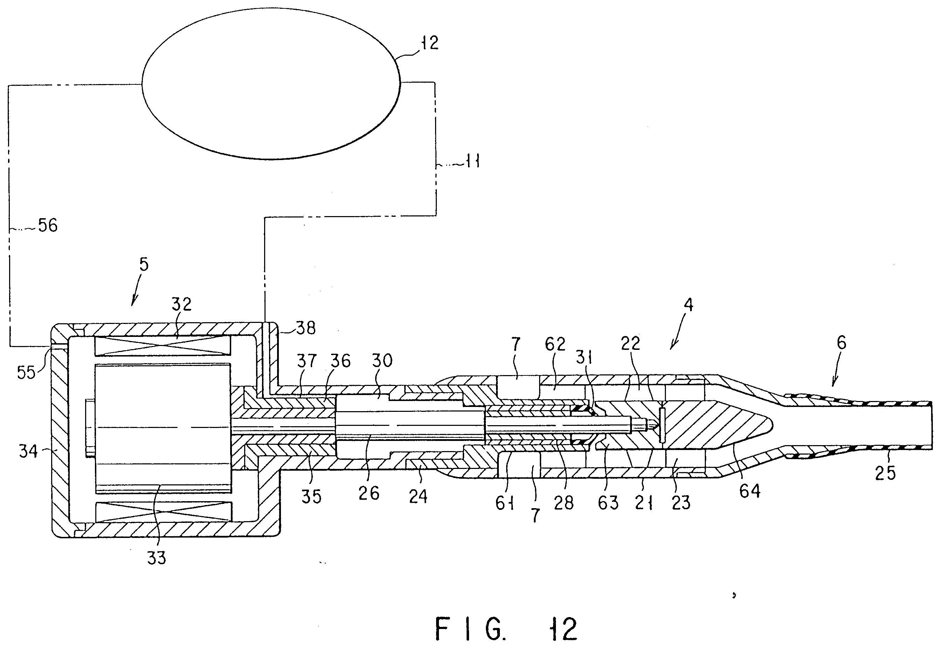

The embodiments of this invention will be described with reference to the accompanying drawings. Fig. 6 and 11 as well as the related description parts are not covered by the present invention, but should improve its understanding.

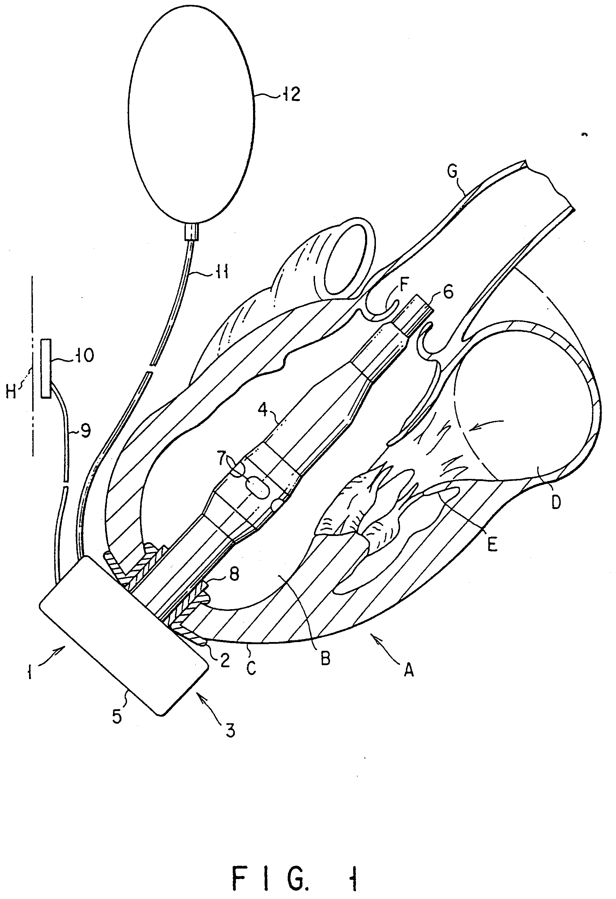

A first embodiment of this invention is shown in FIGS. 1 to 5. FIG. 1 shows an artificial heart of this invention in a state embedded in the left ventricle B of the heart A of a patient (hereinafter referred to as the "human heart"). A cardiac apex, a left atrium, a mitral valve, an aorta valve and an aorta are designated by C, D, E, F and G, respectively.

The artificial heart 1 comprises a cardiac valve ring 2 and the main body 3 of the artificial heart. The cardiac valve ring 2 is a short cylindrical member having a flange and is embedded in the human heart A through the cardiac apex C of the human heart A after the cardiac apex C has been cut. The main body 3 of the artificial heart 1 comprises a pump section 4, a nozzle section 6 provided on the distal end of the pump section 4 and a driving section 5 provided on the proximal end of the pump section 4. The pump section 4 and the nozzle section 6 are inserted in the left ventricle B through the cardiac apex ring 2, and the nozzle section 6 is further inserted in the aorta G through the central portion of the aorta valve F. Liquid tightness is ensured between the cardiac apex ring 2 and the main body 3 by means of a ordinary sealing mechanism, for example, a sealing member 8.

The pump section 4 is a relatively small cylindrical member and it has a smaller volume than the volume of the left ventricle B when it contracts most so as not to prevent natural beats of the human heart A. In the pump section 4 is housed a small axial-flow pump which is driven by a motor provided in the driving section 5. The pump section 4 sucks blood from the left ventricle B at a suction port 7 formed in the outer peripheral surface of the pump section 4 and discharges the blood form the distal end of the nozzle section 6 into the aorta G with the aorta valve F bypassed.

The nozzle section 6 passing through the central portion of the aorta valve F is made of soft synthetic resin material such that it does not suppress the function of the aorta valve F and it does not injure the aorta valve F.

The driving section 5 is embedded in a portion of the thorax outside of the human heart A. In the driving section 5 are provided a motor and other elements such as electric cells and electronic control elements if they are required. The driving section 5 is connected by means of electric wires 9 to a non-contact type electrode 10 embedded in a portion of the human body close to his skin H. A necessary electric power is supplied from an external electric source (not shown) to the driving section 5 through the electrode 10.

The driving section 5 is further connected to a sealing liquid bag 12 by a flexible tube 11. The sealing liquid bag 12 is made of flexible material and filled with a sealing liquid such as a physiological sodium chloride solution. The sealing liquid bag 12 is embedded in a suitable portion of the body of the patient, such as the thorax or the abdominal cavity.

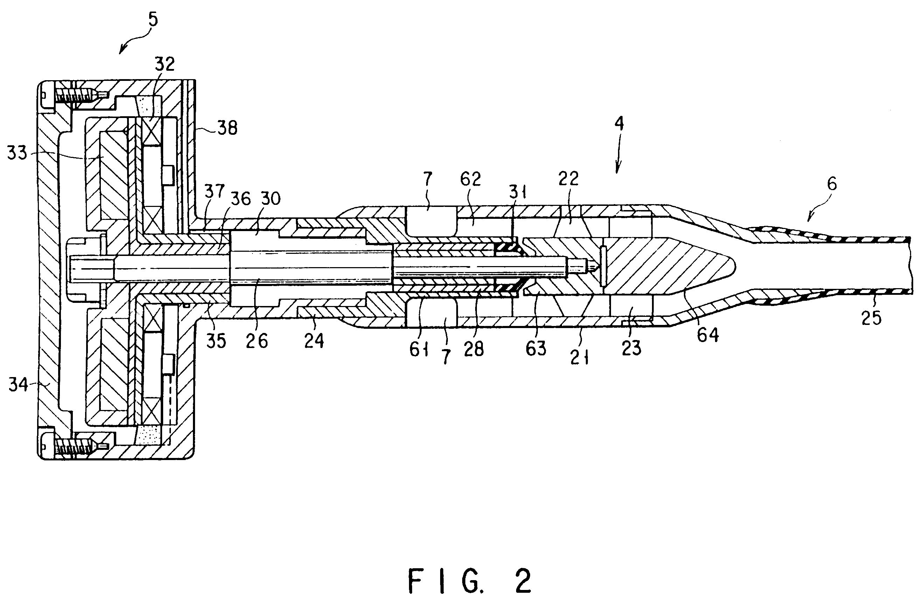

The structure of the main body 3 will be described with reference to FIG. 2. The pump section 4 has a cylindrical casing 21 which is reduced in diameter at its distal end portion to form the nozzle section 6 and is connected at its proximal end portion to the driving section 5 by an extension 24. In the casing 21 are provided an axial-flow pump such as a propeller 22 and a plurality of guide vanes 23 for rectifying a blood flow. The suction port 7 is formed in the outer peripheral surface of the casing 21. In the casing 21 is formed a continuous blood passage extending from the suction port 7 to the nozzle section 6 via the propeller 22 and the guide vanes 23. A thin cylindrical nozzle tube 25 made of flexible synthetic resin material is mounted on the nozzle section 6. As described above, the nozzle tube 25 is designed so as to improve the contacting properties of the nozzle tube 25 with the ventricle valve F, prevent injure of the ventricle valve F and prevent the function of the ventricle valve F form being suppressed. The nozzle tube 25 is a flexible thin cylindrical member. Thus, when the artificial heart happens to be out of order, the nozzle tube 25 is collapsed by the blood pressure in the aorta G so as to act as a check valve for preventing blood from flowing backward from the aorta G to the left ventricle B.

The distal end portion of the driving shaft 26 is connected to the propeller 22. The driving shaft 26 passes through the extension 24 and extends to the interior of the driving section 5. In the driving section 5 is provided a motor having a stator coil 32 and a rotor 33. The proximal end portion of the driving shaft 26 is connected to the rotor 33. A cover 34 seals the driving section 5 hermetically.

In this embodiment, the blood flow passage in the pump section 4 is made smooth. The center portion of the pump section 4 has a boss portion 61, in which the driving shaft 26 and the bearing 28 are inserted. The sealing mechanism 31 is located in the distal end of the boss section 61, and has a conical oil seal. The oil seal is fitted in the conical depression formed in the rear end of a propeller boss 63 of the propeller 22, with a small gap formed between the oil seal and the conical depression. The guide vane 23 has a boss 64 which opposes the front end of the propeller boss 63 and is located at a short distance therefrom. No steps are made between the inner peripheral surface of the casing 21 and the outer surfaces of the boss section 61, propeller boss 63 and boss 64. A smooth annular blood flow passage is therefore formed between the inner peripheral surface of the casing 21 and the outer surface so the components 61, 63 and 64.

The suction port 7 is formed in the proximal part of the boss section 61. A plurality of flow regulating vanes 62 are arranged between the suction port 7 and the propeller 22.

Since the blood flow passage is smooth as described above, the blood can flow smoothly, enhancing the pump efficiency and reducing the number of places where the blood does not flow. Hence, the possibility of thrombi is decreased.

The distal end portion of the driving shaft 26 is supported by a bearing 28 made of ceramic material, and the proximal end portion of the driving shaft 26 is supported by dynamic pressure bearings 35 and 36 made of ceramic material in the driving section 5. On the distal end portion of the driving shaft 26 is provided a sealing mechanism 31 for performing sealing between the interior of the pump section 4 and the interior of the driving section 5 such that blood in the pump section 4 is prevented from entering the driving section 5.

In the portion of the main body 3 between the sealing mechanism 31 and the driving section 5 (for example, the extension 24 of the casing 21) is formed a sealing liquid chamber 30 surrounding the driving shaft 26. A sealing liquid such as a physiological sodium chloride solution is filled in the sealing liquid chamber 30. If necessary, a blood anticoagulant such as heparin or any other chemical agents, if required are added to the sodium chloride solution.

The sealing liquid is not limited to physiological chloride solution. Heparin may be used as sealing liquid.

Between the outer peripheral surface of the dynamic pressure bearing 35 and the inner peripheral surface of the casing 31 is formed an axial groove 37 which communicates with the sealing liquid chamber 30 and a narrow passage 38 formed in the inside wall of the driving section 5. The sealing liquid chamber 30 communicates with the sealing liquid bag 12 via the groove 37, the passage 38 and the tube 11.

The sealing mechanism 31 will be described. In this embodiment, an oil seal is used as the oil seal 31. The oil seal is made of elastic material such as synthetic rubber, and has an elastically deformable lip portion which abuts against the outer peripheral surface of the driving shaft 26 to maintain liquid tightness. The oil seal has a specific feature in that a lubricating film of the sealing liquid is formed between the oil seal itself and the drive shaft 26. This lubricating film ensures the sealing and prevents direct contact of the oil seal with the outer peripheral surface of the driving shaft 26 such that the oil seal is free from wear for a long time.

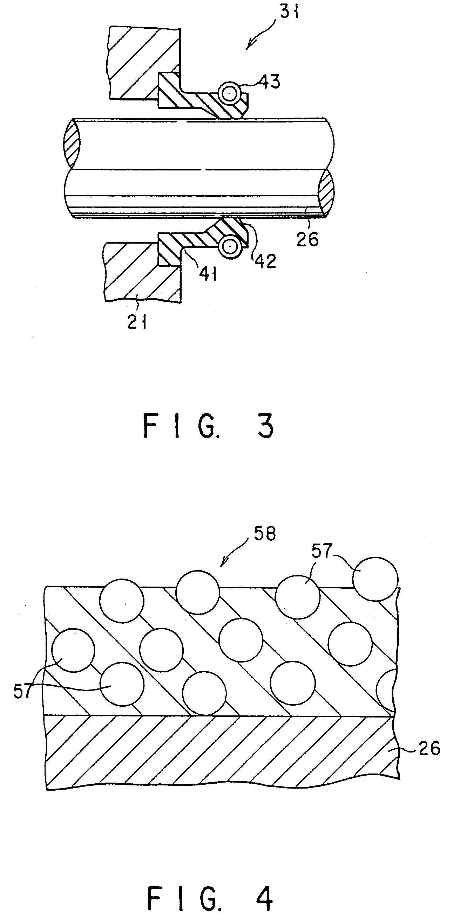

In FIG. 3 is shown an preferred embodiment of the seal mechanism 31 provided with an oil seal 41 made of synthetic rubber, for example, and having a elastically deformable lip portion 42. The inner peripheral surface of the lip portion 42 is in contact with the outer peripheral surface of the driving shaft 26. A garter spring 43 is provided on the oil seal, for stabilizing contact pressure between the lip portion 42 and the outer peripheral surface of the driving shaft 26. As described above, the oil seal 41 is designed such that a thin lubricating film of the sealing liquid is formed between the inner peripheral surface of the lip portion 42 and the outer peripheral surface of the driving shaft 26. The sealing liquid acting as the lubricating film is adapted to circulate on the sealing surface by the rotation of the driving shaft 26. Further, in this embodiment, the sealing liquid acting as the lubricating film is adapted to flow in only one direction toward the pump section 4 such that the sealing liquid is made to flow little by little through the pump section 4. This behavior of the sealing liquid securely prevents blood from entering, from the pump section 4, the sealing surface defined between the oil seal 41 and the driving shaft 26.

Since the sealing liquid is a sodium chloride solution and a very small amount of the solution flows into the pump section 4, it does not affect the human body any more. It is sufficient that the amount of the flowing-out sealing liquid is several cubic centimeters per month, for example, and the amount of the sealing liquid corresponding to the flowing-out amount is supplied form the sealing liquid bag 12. When, therefore, several ten cubic centimeters of the sealing liquid is contained in the sealing liquid bag 12, it is unnecessary to supplement the sealing liquid more than a year.

The oil seal 41 may be made of various materials. Examples of the materials are: silicone rubber, urethane rubber, ethylene-propyrene rubber, nitrile rubber, fluororubber, acrylic rubber, natural rubber, fluororesin, polytetrafluoroethylene, polyurethane, and the like.

A coating is formed on the outer peripheral surface of the driving shaft 26 in order to improve lubricating properties between the oil seal 41 and the driving shaft 26 and enhancing durability. The coating will be described with reference to FIG. 4.

The oil seal 41 is elastically closely fitted on the outer peripheral surface of the driving shaft 26 to prevent blood entrance. Surface treatment is required on the driving shaft 26 so as to maintain the sealing function of the oil seal 41 for a long time. This is because the driving shaft 26 also need to have good lubricating property, wear-resistance, and durability. In this embodiment, a compound plating film is formed on the surface of the driving shaft 26. The compound plating film is formed by making a great number of tetrafluoroethylene fine particles 51 distributed evenly in an electroless nickel plating film so as to eutectic. In the embodiment, the eutectic amount of tetrafluoroethylene is about 25%.

The driving shaft 26 is made of stainless steel, for example. After cleaning (or removing oily material) and activating, nickel is flush-plated on the driving shaft 26. Thereafter, a nickel-plated film having a thickness of several micrometers is formed in a nickelphosphorus plating solution in which there are distributed tetrafluoroethylene fine particles which have been made hydrophilic by a surface active agent. By this treatment, fine particles 57 of tetrafluoroethylene are made eutectic in the nickel plating film, and a compound plating film 58 is formed as shown in FIG. 4.

When the base material of the driving shaft 26 is hardened by heat treatment, the compound plating film provided Vickers hardness of 500 to 600. Since fine particles 57 of tetrafluoroethylene are exposed on the surface of the compound plating film 58, they provide good lubricating properties and water-repellent properties. Fine particles 57 of tetrafluoroethylene is suited for human bodies well. The fine particles 57 are held in the nickel plating film and are combined together. Thus, the fine particles 57 are held firmly so as not to fall of the plating film.

The compound plating film 58 is water-repellent, preventing coagulation of blood, and compatible with living body. The film 58 may be formed not only on the outer peripheral surface of the driving shaft 26 which contacts the oil seal 41, but also on the other components of the artificial heart which contact blood and/or other body fluids.

The sealing mechanism 31 is not limited to an oil seal but can be applicable to the other sealing mechanism such as a labyrinth packing, if the conditions allow.

A dynamic pressure bearing assembly will be described with reference to FIG. 5. The dynamic pressure bearing assembly rotatably supports the proximal end portion of the driving shaft 26 and performs sealing between the sealing liquid chamber 30 and the space in the driving section 5, as well.

The dynamic pressure bearing assembly comprises an outer fixed-side bearing 35 and an inner rotary-side bearing 36 which are made of ceramic material. The fixed-side bearing 35 and the rotary-side bearing 36 have cylindrical portions 35a and 36a and flange portions 35b and 36b, respectively. The cylindrical portion 36a of the rotary-side bearing 36 is closely fitted in the cylindrical portion 35a of the fixed-side bearing 35, and the flange portions 35a and 35b are in a close contact with each other.

A plurality of dynamic-pressure generating grooves 51 and 52 are formed in the outer peripheral surface of the cylindrical portion 36a of the rotary-side bearing 36 and the contacting surface of its flange portion 36b.

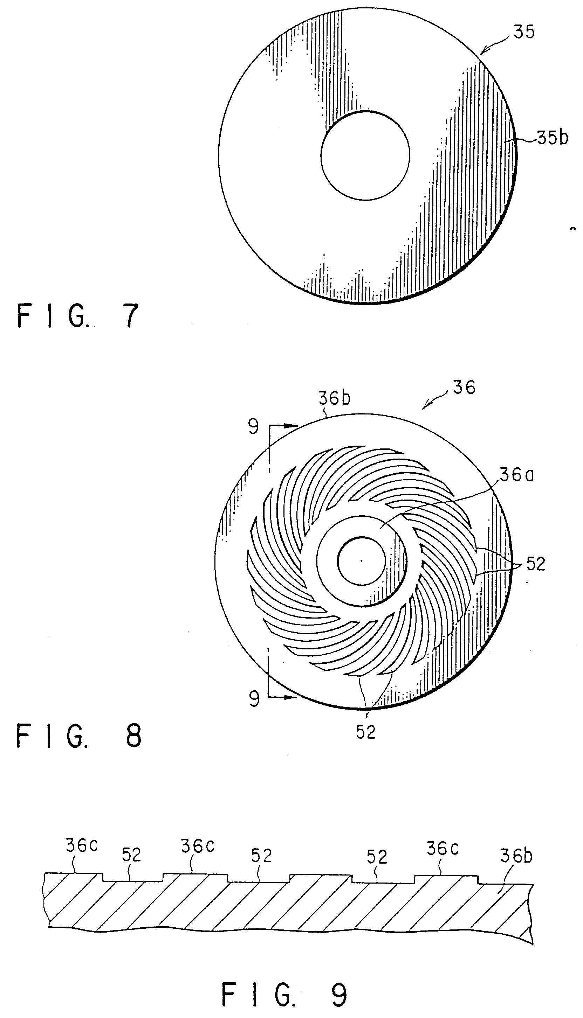

The abutting surface of the flange portion 35b of the fixed-side bearing 35 is flat as illustrated in FIG. 7. A plurality of dynamic-pressure generating grooves 52 are formed in the abutting surface of the flange portion 36b of the rotary-side bearing 36 as shown in FIG. 8. The grooves 52 are curving as shown in FIG. 8. They are shallow as illustrated in FIG. 9, about 3 to 10 microns deep. Those portions of the surface, or the lands 36c located among the grooves 52, and the surface of the flange portion 35b of the fixed-side bearing 35 are smooth, having undulation of 0.3 microns or less and maximum roughness of about 0.1 micron. The dynamic-pressure generating grooves 52 have been formed by performing shot blasting on the abutting surface of the flange portion 36b.

A pair of dynamic-pressure generating grooves 51 are formed in the outer peripheral surface of the cylindrical portion 36a of the rotary-side bearing 36. These grooves 51 are shaped like a herringbone and have the same depth as the dynamic-pressure generating grooves 52 formed in the abutting surface of the flange portion 36b. The grooves 51 extend in the opposite directions. Hence, when the driving shaft 26 is rotated, they guide the sealing liquid in the opposite directions.

The operation of the dynamic pressure bearings 35 and 36 will be described.

When the driving shaft 25 is rotated, the rotary-side bearing 36 is rotated, too. The sealing liquid is thereby supplied from the dynamic-pressure generating grooves 52 toward the center of the flange portion 36b, guided along the dynamic-pressure generating grooves 52 formed in the abutting surface of the flange portion 36b. The sealing liquid is simultaneously supplied to the middle portion of the cylindrical portion 36a, also along the dynamic-pressure generating grooves 51.

As a result, a layer of the sealing liquid is formed under high pressure at the center of the flange portion 36b. Located between the fixed-side bearing 35 and the rotary-side bearing 36, the sealing liquid layer prevents a mechanical contact between the bearings 35 and 36. It serves as lubricant, enabling the rotary-side bearing 36 to rotate with an extremely low resistance applied to it, and preventing wear of the bearing 36. The cylindrical portion 36a and flange portion 36b of the rotary-side bearing 36 bear the radial load and thrust load exerted by the driving shaft 26, respectively.

Since the dynamic-pressure generating grooves 51 and 52 guide the sealing liquid under high pressure to the center of the flange portion 36b and the middle portion of the cylindrical portion 36a, the liquid reliably serves as lubricant though it is material poor in lubrication action.

Both the fixed-side bearing 35 and the rotary-side bearing 36 are made of hard ceramic material such as sintered SiC, sintered α-SiC containing BeO, or sintered Si₃N₄. The ceramic materials exemplified are very hard and have a small coefficient of friction. No wear occurs between the bearings 35 and 36 even if they directly contact each other when the motor is started or stopped or while the motor shaft is rotating.

In FIG. 6 is shown a bearing 28 which comprises a rotary-side bearing 28a and a fixed-side bearing 28b. A pair of dynamic-pressure grooves 70 similar to the dynamic-pressure grooves 51 are formed in the outer peripheral surface of the rotary-side bearing 28a. By the paired dynamic-pressure grooves 70, the sealing liquid is delivered in the opposite directions and a sealing liquid film is formed between the outer peripheral surface of the rotary-side bearing 28a and the inner peripheral surface of the fixed-side bearing 28b. The sealing liquid film reduces resistance of rotation, prevents wear and improves durability.

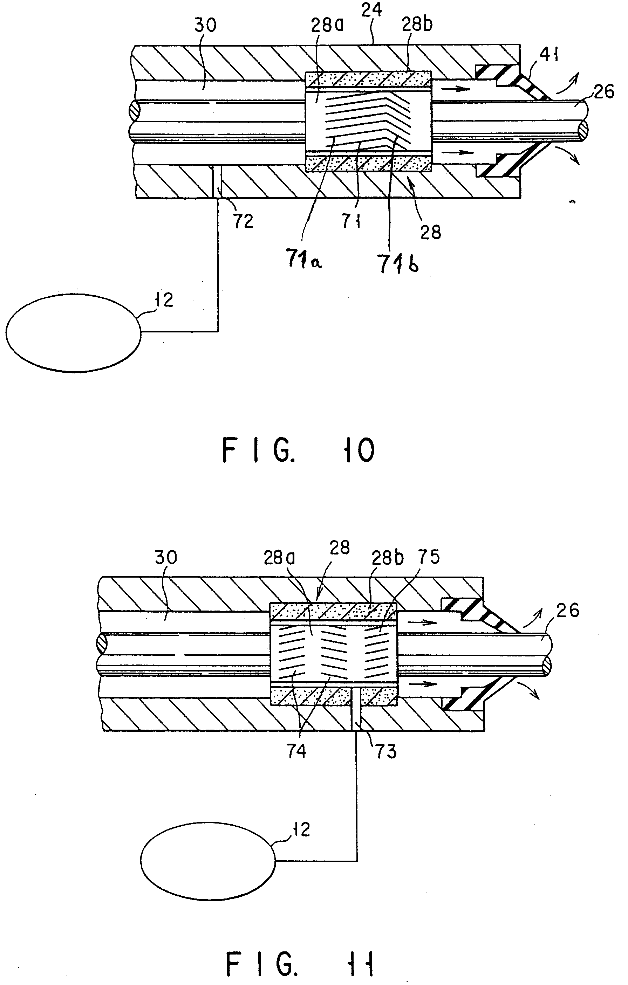

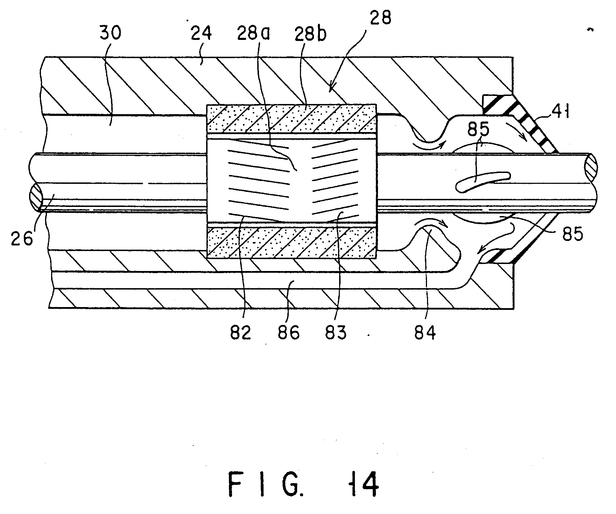

However, according to the invention, the bearing 28 at the distal end of the driving shaft 26 has a structure as shown in FIG. 10 or 14. According to Fig. 10 a plurality of dynamic-pressure generating grooves 71 are formed in the outer peripheral surface of the rotary-side bearing 28a. Each of these grooves 71 consists of two herring-bone-shaped grooves 71a and 71b which extend in different directions. The groove 71a is longer than the groove 71b. The dynamic pressure generated in the dynamic-pressure generating grooves 71 forms a sealing liquid film between the rotary-side bearing 28a and the fixed-side bearing 28b and delivers the sealing liquid toward the oil seal 41. The delivered sealing liquid leaks little by little from between the oil seal 41 and the driving shaft 26 so as to ensure prevention of blood from entering the driving section.

Therefore, the bearing 28 functions not only as a bearing but also as a micropump for supplying the sealing liquid to the oil seal in tiny amounts.

In FIG. 11 is shown another bearing 28 formed in its outer surface with a pair of groups of dynamic-pressure generating grooves and a series of dynamic-pressure generating grooves 75. The paired groups of dynamic-pressure generating grooves 74 deliver the sealing liquid in the opposite directions to form a sealing liquid film between the rotary-side bearing 28a and the fixed-side bearing 28b. The dynamic-pressure generating grooves 75 act to deliver the sealing liquid toward the oil seal 41. The sealing liquid is supplied from the supplying port 73 to the portion between the dynamic-pressure generating grooves 74 and 75.

A second embodiment of this invention will be descried with reference to FIG. 12. The motor housed in the driving section 5 in this embodiment is of an immersed type in a liquid so as to circulate a sealing liquid.

The space in the driving section 5 in this embodiment is filled with the sealing liquid, and the stator 32 and the rotor 33 of the motor are immersed in the sealing liquid. In the proximal end portion of the driving section 5 is formed a passage 55 which is connected to a sealing liquid bag 12 via a pipe 56. In this embodiment, the dynamic pressure bearings 35 and 36 do not perform sealing but act as pumps for supplying the sealing liquid in the driving section 5 toward the sealing liquid chamber 30.

In this embodiment, the sealing liquid circulates by the pump action due to the dynamic pressure generated by the dynamic pressure bearings 35 and 36 in such a manner that the sealing liquid is supplied from the interior of the driving section 5 to the sealing liquid chamber 30 then to the sealing liquid bag 12 through the groove 37, the passage 38 and the pipe 11 and returns to the interior of the driving section 5 through the pipe 56 and the passage 55.

Since the interior of the driving section 5 of this embodiment is filled with the sealing liquid, the motor resistance increases. However, it is unnecessary to consider the possibility that the sealing liquid enters the driving section 5 to make it out of order in this embodiment as it does in the first embodiment, leading to an easy design and enhancing reliability. Because of the circulation of the sealing liquid, the liquid carries heat generated in the stator coil 32 of the motor or the like to the sealing liquid bag such that the heat can be dissipated in the human body in a dispersed manner. Thus, the temperature of the sealing liquid and the driving section 5 is maintained substantially as high as the temperature of the human body. It is unnecessary, therefore, to consider the possibility of low-temperature burn even if the temperature of the surface of the driving section 5 rises more than the temperature of the human body. This makes the design consideration on heat radiation of artificial hearts simple and enhances its reliability. The other structures of the second embodiment are the same as those of the first embodiment. The parts and elements of the second embodiment which are the same as those of the first embodiment are designated by the same reference numerals and the description thereof is omitted.

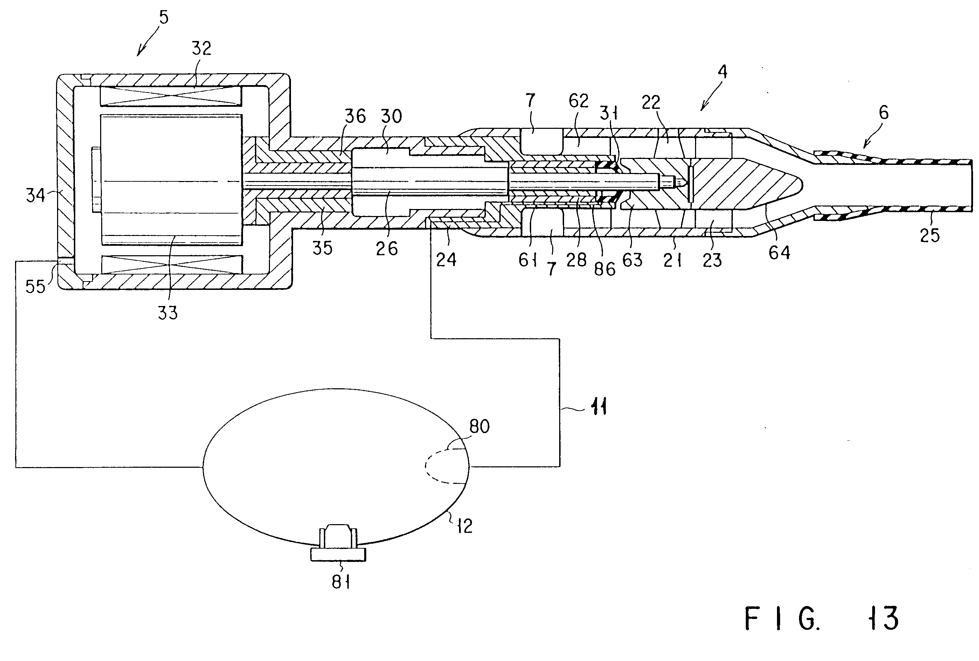

A third embodiment of this invention is shown in FIGS. 13 and 14. Similarly to the second embodiment as shown in FIG. 12, the artificial heart of this embodiment is of a type for circulating a sealing liquid through the main body of the artificial heart and a sealing liquid bag 12. The sealing liquid flows from the supplying port 55 formed in the rear end portion of the driving section 5 into the main body of the artificial heart and then flows out from a vicinity of the sealing mechanism 31 into the sealing liquid bag 12. In this way, the circulation of the sealing liquid is performed. In this embodiment, therefore, the sealing liquid circulates in the overall main body of the artificial heart, whereby the interior of the main body is always maintained clean, leading to high reliability.

A filter 80 is provided in the sealing liquid bag 12, for removing foreign matters contained in the sealing liquid returned from the main body of the artificial heart such that the sealing liquid is always maintained clean. The sealing liquid bag 12 is provided with a liquid therapy port 81 through which the sealing liquid is supplemented or replaced.

FIG. 14 is an enlarged partial view of the sealing mechanism 31 of the main body of the artificial heart of this embodiment. A pair of groups of dynamic pressure grooves 82 and 83 are formed in the outer peripheral surface of the rotary-side bearing 28a of the bearing assembly 28. One group of dynamic pressure grooves 82 is longer in the axial direction than the other group of dynamic pressure grooves 83 such that the former group delivers more sealing liquid than the other group. With this structure, therefore, the sealing liquid is sent in the opposite directions by the dynamic pressure grooves 82 and 83 and a sealing liquid film is formed between the rotary-side bearing 28a and the fixed-side bearing 28b due to the dynamic pressure in the dynamic pressure grooves 82 and 83. Since the former group of dynamic pressure grooves 82 deliver more sealing liquid than the latter group of dynamic pressure grooves 83 do, the sealing liquid is supplied toward the oil seal 41.

When the sealing liquid which is being sent toward the oil seal 41 passes through the orifice portion 84, the flow speed of the liquid increases and is sent to a rear side portion of the oil seal 41. Vane projections 85 are formed on the portion of the driving shaft 26 which is close to the oil seal 41. As the driving shaft 26 rotates, the vane projections 85 agitate the sealing liquid therearound and deliver it to the rear side portion of the oil seal 41. As a result, neither the sealing water nor foreign matters stay in the rear side portion of the oil seal 41, whereby sealing of the oil seal 41 is securely maintained.

In the wall of the extension 24 of the casing 21 of the main body of the artificial heart is formed an exhausting passageway 86 through which the sealing liquid in the rear side portion of the oil seal 41 is sent to the sealing liquid bag 12.

## Claims

1. An artificial heart including a main body (3) of said artificial heart (1) comprising:
a cylindrical pump section (4);
a driving section (5) having a driving shaft (26) and being provided on a proximal end of said pump section (4) for driving said pump section (4) via said driving shaft (26), said driving shaft (26) having an outer peripheral surface;
a sealing mechanism (31) provided between said driving section (5) and said pump section (4), for maintaining said driving shaft (26) in a liquid tight state and preventing blood from entering said driving section (5) from said pump section (4);
characterized by
a sealing liquid chamber (30) surrounding said driving shaft (26) between said sealing mechanism (31) and said driving section (5), said sealing liquid chamber (30) being filled with a sealing liquid;
a sealing liquid bag (12) filled with said sealing liquid, said sealing liquid chamber (30) communicating with said sealing liquid bag (12); and
a dynamic-pressure bearing (28) provided at a distal end portion of said driving shaft (26), said dynamic-pressure bearing (28) functioning as micropump for supplying the sealing liquid to said sealing mechanism (31).

2. The artificial heart according to claim 1, characterized in that said sealing mechanism (31) has an oil seal (41) made of elastic material closely fitted on said outer peripheral surface of said driving shaft (26) and formed with a lubricating film of said sealing liquid between said oil seal (41) and said outer peripheral surface of said driving shaft (26).

3. The artificial heart according to claim 1 or 2, characterized in that said outer peripheral surface of said driving shaft (26) is covered with a coating film comprising plated metal and fine particles made of tetrafluoroethylene and derivatives thereof distributed in said plated metal.

4. The artificial heart according to one of claims 1 to 3, characterized in that said sealing liquid bag (12) is embeddable in a human body.

5. The artificial heart according to one of claims 1 to 4, characterized in that said driving section (5) is provided with a dynamic-pressure bearing (35, 36) for supporting said driving shaft (26) and delivering said sealing liquid toward said sealing liquid chamber (30) by a dynamic pressure generated by rotation of said dynamic-pressure bearing (35, 36), said dynamic-pressure bearing (35, 36) is provided between the driving section (5) and the sealing liquid chamber (30).

6. The artificial heart according to one of claims 1 to 4, characterized in that said driving section (5) is provided with a dynamic-pressure bearing (35, 36) for supporting said driving shaft (26) and for maintaining said driving section (5) in a liquid tight state, said sealing liquid chamber (30) is formed between the dynamic-pressure bearing (35, 36) and said sealing mechanism (31), said driving section (15) is filled with a gas, and said dynamic-pressure bearing (35, 36) is provided between the driving section (5) and the sealing liquid chamber (30).

7. The artificial heart according to claim 5 or 6, characterized in that said dynamic-pressure bearing (35, 36) is made of ceramic material.

8. The artificial heart according to one of claims 5 to 7, characterized in that said dynamic-pressure bearing (35, 36) comprises a rotary-side unit (36) and a fixed-side unit (35), each having a cylindrical portion (36a, 35a) for receiving radial load and a flange portion (36b, 35b) for receiving thrust load, and dynamic-pressure generating grooves (51, 52) are formed in the cylindrical and flange portions (36a, 36b) of at least said rotary-side unit (36).

9. The artificial heart according to one of claims 1 to 8, characterized in that said dynamic-pressure bearing (28) at the distal end of the driving shaft (26) is made of a ceramic material.

10. The artificial heart according to one of claims 1 to 9, characterized in that said dynamic-pressure bearing (28) at the distal end of the driving shaft (26) comprises a cylindrical portion (28a) for receiving radial load, and dynamic-pressure generating grooves (70, 71, 74, 75) are formed in an outer peripheral surface of the cylindrical portion (28a) of said dynamic-pressure seal bearing (28).

11. The artificial heart according to one of claims 1 to 5, 7 to 10, characterized in that said driving section (5) has an interior filled with said sealing liquid, and said sealing liquid chamber (30) extends to said interior of said driving section.

12. The artificial heart according to one of claims 1 to 11, characterized by further comprising vane projections (85) formed adjacent to said sealing mechanism (31) on said outer peripheral surface of said driving shaft (26), for agitating said sealing liquid therearound.

## Patentansprüche

1. Künstliches Herz mit einem Hauptkörper (3) des künstlichen Herzens (1):
mit einem zylindrischen Pumpenabschnitt (4);
mit einem Treiberabschnitt (5) mit einer Antriebswelle (26), welche an einem nahen Ende des Pumpenabschnittes (4) angeordnet ist, um den Pumpenabschnitt (4) durch die Antriebswelle (26) anzutreiben, wobei die Antriebswelle (26) eine äußere Umfangsfläche aufweist;
mit einem Dichtungsmechanismus (31) zwischen dem Treiberabschnitt (5) und dem Pumpenabschnitt (4), um die Antriebswelle (26) in einem flüssigkeitsdichten Zustand zu halten und ein Eindringen von Blut in den Treiberabschnitt (5) vom Pumpenabschnitt (4) zu verhindern;
gekennzeichnet durch
eine Dichtungsflüssigkeits-Kammer (30), welche die Antriebswelle (26) zwischen dem Dichtungsmechanismus (31) und dem Treiberabschnitt (5) umgibt, wobei die Dichtungsflüssigkeits-Kammer (30) mit einer Dichtungsflüssigkeit gefüllt ist;
eine Dichtungsflüssigkeits-Tasche (12), welche mit der Dichtungsflüssigkeit gefüllt ist, wobei die Dichtungsflüssigkeits-Kammer (30) mit der DichtungsflüssigkeitsTasche (12) verbunden ist; und
ein dynamisches Drucklager (28) an einem entfernten Endbereich der Antriebswelle (26), wobei das dynamische Drucklager (28) als Mikropumpe zum Zuführen der Dichtungsflüssigkeit zum Dichtungsmechanismus (31) dient.

2. Künstliches Herz nach Anspruch 1, dadurch gekennzeichnet, daß der Dichtungsmechanismus (31) eine Öldichtung (41) aus elastischem Material aufweist, welche auf die äußere Umfangsfläche der Antriebswelle (26) eng aufgepaßt ist und mit einem Schmiermittelfilm aus Dichtungsflüssigkeit zwischen der Öldichtung (41) und der äußeren Umfangsfläche der Antriebswelle (26) ausgebildet ist.

3. Künstliches Herz nach Anspruch 1 oder 2, dadurch gekennzeichnet, daß die äußere Umfangsfläche der Antriebswelle (26) mit einem Beschichtungsfilm bedeckt ist, welcher plattiertes Metall und feine Partikel aus Tetrafluoroethylen und dessen Derivate aufweist, die in dem plattierten Metall verteilt sind.

4. Künstliches Herz nach einem der Ansprüche 1 bis 3, dadurch gekennzeichnet, daß die Dichtungsflüssigkeits-Tasche (12) im menschlichen Körper implantierbar ist.

5. Künstliches Herz nach einem der Ansprüche 1 bis 4, dadurch gekennzeichnet, daß der Treiberabschnitt (5) ein dynamisches Drucklager (35, 36) zum Stützen der Antriebswelle (26) und zum Zuführen der Dichtungsflüssigkeit zur Dichtungsflüssigkeits-Kammer (30) durch den dynamischen Druck aufweist, welcher durch die Rotation des dynamischen Drucklagers (35, 36) erzeugt wird, wobei das dynamische Drucklager (35, 36) zwischen dem Treiberabschnitt (5) und der Dichtungsflüssigkeits-Kammer (30) angeordnet ist.

6. Künstliches Herz nach einem der Ansprüche 1 bis 4, dadurch gekennzeichnet, daß der Treiberabschnitt (5) mit einem dynamischen Drucklager (35, 36) zum Stützen der Antriebswelle (26) und zum Beibehalten des Treiberabschnittes (5) in einem flüssigkeitsdichten Zustand angeordnet ist, wobei die Dichtungsflüssigkeits-Kammer (30) zwischen dem dynamischen Drucklager (35, 36) und dem Dichtungsmechanismus (31) ausgebildet ist, der Treiberabschnitt (5) mit Gas gefüllt ist, und das dynamische Drucklager (35, 36) zwischen dem Treiberabschnitt (5) und der Dichtungsflüssigkeits-Kammer (30) angeordnet ist.

7. Künstliches Herz nach Anspruch 5 oder 6, dadurch gekennzeichnet, daß das dynamische Drucklager (35, 36) aus Keramikmaterial besteht.

8. Künstliches Herz nach einem der Ansprüche 5 bis 7, dadurch gekennzeichnet, daß das dynamische Drucklager (35, 36) eine drehseitige Einheit (36) und eine fixierseitige Einheit (35) aufweist, die jeweils einen zylindrischen Bereich (36a, 35a) zur Aufnahme einer Radiallast und einen Flanschbereich (36b, 35b) zur Aufnahme einer Schublast, sowie dynamischen Druck erzeugende Nuten (51, 52) aufweisen, welche im zylindrischen Bereich und Flanschbereich (36a, 36b) der zumindest drehseitigen Einheit (36) ausgebildet sind.

9. Künstliches Herz nach einem der Ansprüche 1 bis 8, dadurch gekennzeichnet, daß das dynamische Drucklager (28) am entfernten Ende der Antriebswelle (26) aus Keramikmaterial besteht.

10. Künstliches Herz nach einem der Ansprüche 1 bis 9, dadurch gekennzeichnet, daß das dynamische Drucklager (28) am entfernten Ende der Antriebswelle (26) einen zylindrischen Bereich (28a) zur Aufnahme einer Radiallast umfaßt, und daß dynamischen Druck erzeugende Nuten (70, 71, 74, 75) in der äußeren Umfangsfläche des zylindrischen Bereiches (28a) des dynamischen Drucklagers (28) ausgebildet sind.

11. Künstliches Herz nach einem der Ansprüche 1 bis 5 und 7 bis 10, dadurch gekennzeichnet, daß der Treiberabschnitt (5) einen mit der Dichtungsflüssigkeit gefüllten Innenraum aufweist, und daß die Dichtungsflüssigkeits-Kammer (30) zum Innenraum des Treiberabschnittes verläuft.

12. Künstliches Herz nach einem der Ansprüche 1 bis 11, gekennzeichnet durch Flügelvorsprünge (85), welche benachbart dem Dichtungsmechanismus (31) auf der äußeren Umfangsfläche der Antriebswelle (26) ausgebildet sind, um die Dichtungsflüssigkeit um die Antriebswelle zu führen.

## Revendications

1. Coeur artificiel comportant un corps principal (3) dudit coeur artificiel (1) comprenant :
une section de pompe cylindrique (4);
une section d'entraînement (5) ayant un arbre d'entraînement (26) et étant prévue sur une extrémité proximale de ladite section de pompe (4) pour entraîner ladite section de pompe (4) par le biais dudit arbre d'entraînement (26), ledit arbre d'entraînement (26) ayant une surface périphérique externe;
un mécanisme d'étanchéité (31) prévu entre ladite section d'entraînement (5) et ladite section de pompe (4), pour maintenir ledit arbre d'entraînement (26) dans un état étanche aux liquides et pour empêcher que du sang ne pénètre dans ladite section d'entraînement (5) depuis ladite section de pompe (4);
caractérisé par
une chambre (30) de liquide obturant entourant ledit arbre d'entraînement (26) entre ledit mécanisme d'étanchéité (31) et ladite section d'entraînement (5), ladite chambre (30) de liquide obturant étant remplie d'un liquide obturant;
un sac (12) de liquide obturant rempli dudit liquide obturant, ladite chambre (30) de liquide obturant communiquant avec ledit sac (12) de liquide obturant; et
un palier de pression dynamique (28) prévu au niveau d'une portion d'extrémité distale dudit arbre d'entraînement (26), ledit palier de pression dynamique (28) fonctionnant en tant que micropompe pour alimenter ledit mécanisme d'étanchéité (31) en liquide obturant.

2. Coeur artificiel selon la revendication 1, caractérisé en ce que ledit mécanisme d'étanchéité (31) a un joint étanche à l'huile (41) en matériau élastique monté à ajustement serré sur ladite surface périphérique externe dudit arbre d'entraînement (26) et formé avec une pellicule lubrifiante dudit liquide obturant entre ledit joint étanche à l'huile (41) et ladite surface périphérique externe dudit arbre d'entraînement (26).

3. Coeur artificiel selon la revendication 1 ou 2, caractérisé en ce que ladite surface périphérique externe dudit arbre d'entraînement (26) est recouverte d'une pellicule de revêtement comprenant un placage métallique et de fines particules en tétrafluoroéthylène et en dérivés de celui-ci, réparties dans ledit placage métallique.

4. Coeur artificiel selon l'une quelconque des revendications 1 à 3, caractérisé en ce que ledit sac (12) de liquide obturant peut être implanté dans un corps humain.

5. Coeur artificiel selon l'une quelconque des revendications 1 à 4, caractérisé en ce que ladite section d'entraînement (5) est pourvue d'un palier de pression dynamique (35, 36) pour supporter ledit arbre d'entraînement (26) et pour alimenter ladite chambre (30) de liquide obturant en liquide obturant par pression dynamique générée par la rotation dudit palier de pression dynamique (35, 36), ledit palier de pression dynamique (35, 36) étant prévu entre la section d'entraînement (5) et la chambre (30) de liquide obturant.

6. Coeur artificiel selon l'une quelconque des revendications 1 à 4, caractérisé en ce que ladite section d'entraînement (5) est pourvue d'un palier de pression dynamique (35, 36) pour supporter ledit arbre d'entraînement (26) et pour maintenir ladite section d'entraînement (5) dans un état étanche aux liquides, ladite chambre (30) de liquide obturant est formée entre le palier de pression dynamique (35, 36) et ledit mécanisme d'étanchéité (31), ladite section d'entraînement (5) est remplie d'un gaz, et ledit palier de pression dynamique (35, 36) est prévu entre la section d'entraînement (5) et la chambre (30) de liquide obturant.

7. Coeur artificiel selon la revendication 5 ou 6, caractérisé en ce que ledit palier de pression dynamique (35, 36) est en matériau céramique.

8. Coeur artificiel selon l'une quelconque des revendications 5 à 7, caractérisé en ce que ledit palier de pression dynamique (35, 36) comprend une unité du côté rotatif (36) et une unité du côté fixe (35), chacune ayant une portion cylindrique (36a, 35a) pour recevoir l'effort radial et une portion de bride (36b, 35b) pour recevoir la poussée longitudinale, et des rainures (51, 52) générant une pression dynamique sont formées dans les portions cylindrique et de bride (36a, 36b) d'au moins ladite unité du côté rotatif (36).

9. Coeur artificiel selon l'une quelconque des revendications 1 à 8, caractérisé en ce que ledit palier de pression dynamique (28) au niveau de l'extrémité distale de l'arbre d'entraînement (26) est en matériau céramique.

10. Coeur artificiel selon l'une quelconque des revendications 1 à 9, caractérisé en ce que ledit palier de pression dynamique (28) au niveau de l'extrémité distale de l'arbre d'entraînement (26) comprend une portion cylindrique (28a) pour recevoir l'effort radial, et des rainures (70, 71, 74, 75) générant une pression dynamique sont formées dans une surface périphérique externe de la portion cylindrique (28a) dudit palier de pression dynamique (28).

11. Coeur artificiel selon l'une quelconque des revendications 1 à 5 et 7 à 10, caractérisé en ce que ladite section d'entraînement (5) a un intérieur rempli dudit liquide obturant, et ladite chambre (30) de liquide obturant s'étend jusque dans ledit intérieur de ladite section d'entraînement.

12. Coeur artificiel selon l'une quelconque des revendications 1 à 11, caractérisé en ce qu'il comprend en outre des saillies en ailettes (85) formées à proximité dudit mécanisme d'étanchéité (31) sur ladite surface périphérique externe dudit arbre d'entraînement (26) pour agiter ledit liquide obturant autour de celui-ci.
